# EUROPEAN PATENT SPECIFICATION

(11) **EP 2 756 831 B1**
(45) Date of publication and mention of the grant of the patent: **07.08.2019**
(21) Application number: 12832071.0
(22) Date of filing: 07.09.2012
(51) Int. Cl.: A61F 13/15, A61F 13/49, B32B 37/12, B32B 37/20, B32B 38/00, B32B 38/18

(54) **OSCILLATING/FEEDING APPARATUS FOR CONTINUOUS MEMBER**
SCHWINGUNGS-/ZUFÜHRUNGSVORRICHTUNG FÜR KONTINUIERLICHES ELEMENT
APPAREIL D'OSCILLATION/ALIMENTATION POUR UN ÉLÉMENT CONTINU

(30) Priority: 12.09.2011 JP 2011198786
(43) Date of publication of application: 23.07.2014
(73) Proprietor: Kao Corporation, Chuo-ku Tokyo 103-8210 (JP)
(72) Inventor: KOKUBO, Makoto, Haga-gun Tochigi 321-3497 (JP); SERA Takeshi, Haga-gun Tochigi 321-3497 (JP)
(74) Representative: Vossius & Partner Patentanwälte Rechtsanwälte mbB
(86) International application number: PCT/JP2012/072928
(87) International publication number: WO 2013/039004

(56) References cited:
- EP-A1- 2 679 205
- EP-A1- 2 679 206
- JP-A- H0 970 412
- JP-A- H04 325 154
- JP-A- 2001 178 769
- US-A- 5 389 173
- US-A- 5 525 175
- US-A1- 2003 010 423
- US-A1- 2003 120 245
- US-B1- 6 217 690

## Description

### Technical Field

The present invention relates to an oscillating/feeding apparatus for a continuous member.

### Background Art

In the past, in the manufacture of a disposable diaper or the like, in order to dispose an elastic member, which forms gathers, in a curved shape, the elastic member has been fed to a continuously conveyed sheet while being oscillated in a direction intersecting a traveling direction of the sheet.

For example, JP 9-70412 A (Patent Literature 1) discloses an apparatus for forming curved gathers on a paper diaper by an oscillating mechanism using a servo motor. Patent Literature 1 discloses a specific oscillating mechanism which includes a driving pulley, a driven pulley, and a timing belt bridged between the pulleys, and oscillates a guide portion locked to the timing belt by controlling the driving pulley by the servo motor according to specific control data. A hole, which leads an elastic member, is formed at the guide portion. Accordingly, it is possible to dispose the elastic member between upper and lower sheets in a curved shape by leading the elastic member while oscillating the guide portion.

However, when the guide portion for positioning rubber is oscillated by the rotation of the servo motor, the speed-up of a facility or the change of a disposition shape in which the elastic member is disposed is limited by a mechanical condition such as the speed or acceleration of the guide portion. For example, when the speed of the guide portion is increased by the speed-up of the facility or the change of the disposition shape, acceleration is increased proportional to the square of the speed. Accordingly, a motor, which can output large torque corresponding thereto, is required.

Since a motor having large torque has a large inertia, large torque is required to rotate the motor at a high speed. As a result, it is difficult to increase the speed or acceleration of the guide portion, and it may be difficult to cope with the speed-up of the facility or the change of the disposition shape of the elastic member increasing the speed or acceleration of the guide portion in a technique disclosed in Patent Literature 1.

Further, JP 2010-88750 A (Patent Literature 2) discloses a technique that solves the above-mentioned problem by increasing or decreasing the speed of the sheet according to a curve shape of the disposed elastic member, displacing the guide portion according to the speed of the sheet to reduce the speed or acceleration of the guide portion, and reducing the rotational speed or acceleration of the motor when an elastic member is introduced between two sheets while oscillating the elastic member by an oscillating mechanism using a servo motor.

However, when the speed of the sheet to which the elastic member is fixed is increased or decreased, the tension of the sheet is changed and a slack or wrinkles are generated on the sheet. For this reason, there is a concern that a defect occurs in the quality of a finished product, or a trouble such as winding or cutting occurs, which causes the deterioration of productivity.

US 2003/120245 (A1) relates to elastic composites formed within such garments, or formed separately from such garments and secured therein, to provide an elastic component to such garments. US 2003/010423 (A1) relates to an apparatus and method for manufacturing disposable wearing articles such as paper diapers and disposable pants. US 5 389 173 (A) relates to apparatus for making disposable type diaper products and, more particularly, diaper products wherein there is a contoured leg elastic. US 5 525 175 (A) relates to an apparatus and method for applying elastic strands in a curved configuration at each of the leg opening regions of a disposable absorbent article, such as a disposable diaper. US 6 217 690 (B1) relates to women's disposable undergarments having an absorbent layer or fluid repellent region or both to be used with a woman's normal feminine care protection during her menstrual period. EP 2 679 206 (A1) and EP 2 679 205 (A1) relates to an apparatus for manufacturing an absorbent article and a method for manufacturing the absorbent article for arranging an elastic member on a web that is conveyed in a continuous state configuring a part of component of the absorbent article, and a method for manufacturing the absorbent article.

### Summary of Invention

According to the present invention, there is provided an oscillating/feeding apparatus for a continuous member that feeds a continuous member onto a continuously conveyed continuous sheet while oscillating the continuous member in an intersecting direction intersecting a traveling direction of the sheet. The apparatus includes: an oscillating unit that is oscillated in the intersecting direction; a first displacement means that displaces and oscillates the oscillating unit in the intersecting direction; a second displacement means that displaces and oscillates the oscillating unit in the intersecting direction by displacing a part or all of the first displacement means in the intersecting direction; and a control unit that controls the first displacement means and second displacement means. The oscillating unit includes an outlet which feeds the continuous member to the sheet.

Moreover, according to the present invention, there is provided a method of manufacturing a composite sheet in which a continuous member is fixed to a sheet in a curved shape, and the method includes: a feeding step of continuously feeding the continuous member onto a continuously conveyed sheet, wherein in the feeding step, the continuous member is fed while being oscillated in a direction intersecting the traveling direction of the sheet by the oscillating/feeding apparatus for a continuous member.

Meanwhile, the expression of "feeds a continuous member onto a continuous sheet" also includes a case in which a continuous member is fed onto a continuous sheet, and, then, another continuous sheet or the like is joined to the surface of the sheet onto which the continuous member has been fed, a case in which a continuous member is fed onto a continuous sheet, and, at the same time, another continuous sheet or the like is joined to the surface of the sheet onto which the continuous member is fed (a case in which a continuous member is directly introduced to a joint portion between two sheets), and the like.

### Brief Description of Drawings

Fig. 1 is a view illustrating an oscillating/feeding apparatus for an elastic member according to a first embodiment of the invention and an aspect where an extensible sheet (composite sheet) is manufactured using the apparatus.
Fig. 2 is a schematic view of a portion at which elastic members are supplied using the apparatus according to the first embodiment when viewed from the side.
Fig. 3 is a perspective view illustrating an aspect where a continuous outer cover (extensible sheet) of a pull-on disposable diaper is manufactured using the apparatus according to the first embodiment.
Fig. 4 is a graph illustrating an example of a moving distance of an oscillating unit that is caused by the rotation of a belt and a moving distance of the oscillating unit that is caused by the displacement of the belt (the sum of the moving distances is a moving distance of the oscillating unit).
Fig. 5 is a view illustrating another embodiment of the invention.
Fig. 6 is a view illustrating another embodiment of the invention.
Fig. 7 is a view illustrating another embodiment of the invention.
Fig. 8 is a view illustrating another embodiment of the invention.
Fig. 9 is a view illustrating another embodiment of the invention.
Fig. 10 is a view illustrating another embodiment of the invention.
Fig. 11 is a view illustrating a modification of the first embodiment.

### Description of Embodiments

The invention is an oscillating/feeding apparatus according to claim 1, and methods of manufacturing a composite sheet and a diaper by means of this apparatus, according to claims 7 and 12, and will be described on the basis of preferred embodiments thereof with reference to the drawings.

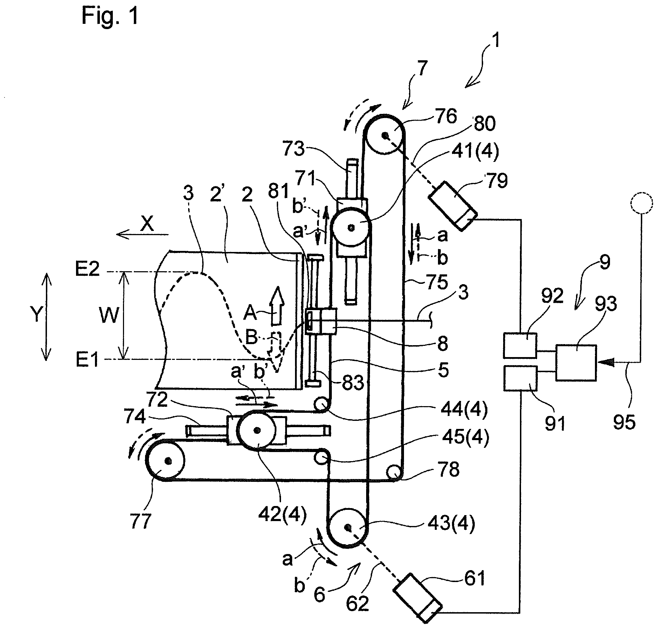

An oscillating/feeding apparatus 1 for an elastic member (oscillating member) according to a first embodiment of the invention is an apparatus that feeds an elastic member 3 onto a continuously conveyed continuous sheet 2 while oscillating the elastic member 3 in an intersecting direction Y intersecting a traveling direction X of the sheet 2 as illustrated in Fig. 1. In Fig. 1, in order to illustrate an aspect of the oscillation of the elastic member 3, the traveling direction (conveying direction) X of the continuous sheet 2 is illustrated as a direction that is directed to the left side from the right side of Fig. 1. However, an actual traveling direction (conveying direction) X is a direction that is substantially perpendicular to the plane of the drawing. Further, an introduction direction of the elastic member 3 is also the same as described above.

The oscillating/feeding apparatus 1 for an elastic member includes a belt rotation means 6 (first displacement means) that rotates an endless first belt 5 bridged between a plurality of pulleys 4, a belt displacement means 7 (second displacement means) that displaces the first belt 5 in the intersecting direction Y intersecting the traveling direction X of the sheet 2, an oscillating unit 8 that is fixed to the first belt 5 and is oscillated in the intersecting direction Y with the rotation and displacement of the first belt 5, and a control unit 9 that controls the rotation and the displacement of the first belt 5. The oscillating unit 8 includes an outlet 81 which feeds the elastic member 3 to the sheet 2.

The oscillating/feeding apparatus 1 for an elastic member according to this embodiment includes the belt rotation means 6 that rotates the first belt 5 and the belt displacement means 7 that displaces the first belt 5.

The first belt 5 is an endless timing belt, and is bridged between a driving pulley 43, driven pulleys 41 and 42 provided on sliders 71 and 72, and immovable driven pulleys 44 and 45. Further, the driving pulley 43 is rotationally driven by a first servo motor 61, so that the first belt 5 can be rotated in a normal direction and a reverse direction. The rotation of the first belt 5 is rotation that also can occur due to the rotation of the driving pulley 43 under a state where the positions of the sliders 71 and 72 are fixed and the positions of all the pulleys are fixed.

The belt rotation means 6 (first displacement means) of this embodiment includes a well-known power transmission mechanism 62 that is formed by the combination or the like of the first servo motor 61 as a driving source and gears transmitting the rotation of the motor 61 to the driving pulley 43.

The belt displacement means 7 (the second displacement means of this embodiment) is a means that moves the position of at least a portion of the first belt 5, to which the oscillating unit 8 is fixed, in the intersecting direction Y intersecting the traveling direction of the sheet 2. As illustrated in Fig. 1, the belt displacement means 7 of the apparatus 1 according to this embodiment includes the sliders 71 and 72 on which the driven pulleys 41 and 42 are rotatably provided, slide guides 73 and 74 that determine directions in which both the sliders 71 and 72 slide, a second belt 75 that is connected to both the sliders 71 and 72, and a driving pulley 76 and driven pulleys 77 and 78 between which the second belt 75 is bridged. The second belt 75 is also a means that connects a plurality of sliders 71 and 72. Further, the driving pulley 76 is rotationally driven by a second servo motor 79, so that the driven pulleys 77 and 78 are driven and rotated. The second belt 75 and the sliders 71 and 72 joined to the second belt 75 are displaced in an a' or b' direction of Fig. 1 with the rotation of these pulleys 76 to 78. It is preferable that the second belt 75 be also a timing belt.

The belt displacement means 7 includes a well-known power transmission mechanism 80 that is formed by the combination or the like of the second servo motor 79 as a driving source and gears transmitting the rotation of the motor 79 to the driving pulley 76.

Since the sliders 71 and 72 are connected to the driven pulleys 41 and 42 that form a part of the belt rotation means 6 (first displacement means), the belt displacement means 7 (second displacement means) of this embodiment displaces and oscillates the oscillating unit 8 in the intersecting direction Y by moving the position of the driven pulley 41, which is a part of the belt rotation means 6 (first displacement means), in the intersecting direction Y. On the other hand, the belt displacement means 7 (second displacement means) of this embodiment does not move the positions of the driving pulley 43, the first servo motor 61 (driving source), the power transmission mechanism 62, and the like of the belt rotation means 6 (first displacement means).

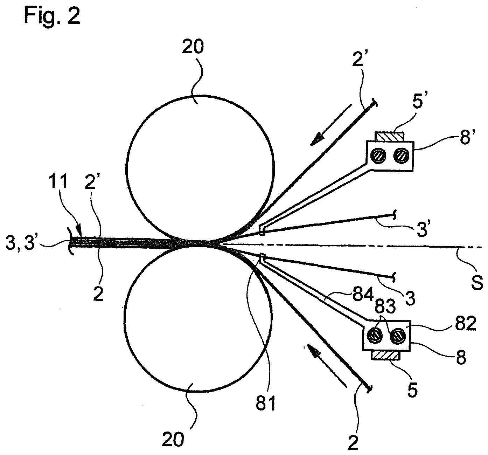

The oscillating unit 8 is fixed to the first belt 5, and is located between the driven pulley 41 and the driven pulley 44. As illustrated in Fig. 2, the oscillating unit 8 includes two through holes that are formed at a block-like fixed part 82 fixed to the first belt 5, and oscillates along two slide shafts 83 that are inserted into the through holes, respectively. The slide shafts 83 of the oscillating unit 8 are disposed along the intersecting direction Y so as to be parallel to each other. The slide shaft 83 is an example of a slide guide that regulates the displacement direction of the above-mentioned sliders 71 and 72, and a slide rail or the like can be used instead of the slide shaft as the slide guide.

The oscillating unit 8 includes an extension portion 84 that obliquely extends from the fixed part 82, and the outlet 81 for the elastic member 3, which includes a guide hole (not illustrated) into which the elastic member 3 is inserted, is formed at a tip portion of the extension portion 84. The outlet 81 for the elastic member may include a guide groove or the like instead of the guide hole. Further, only one elastic member 3 led from the oscillating unit 8 is illustrated in Fig. 1. However, one elastic member or a plurality of elastic members 3 may be oscillated by one oscillating unit 8, and it is preferable that the oscillating unit 8 be provided with guide holes or guide grooves of which the number is the same as the number of elastic members to be oscillated.

The rotation, the stop, the rotational direction, the rotational position, the speed, and the like of each of the first and second servo motors 61 and 79 can be electrically controlled. Accordingly, it is possible to oscillate the oscillating unit 8 in a desired pattern on the basis of a reference signal, which cyclically changes for each product 1, by controlling these by the control unit 9.

Specifically, the control unit 9 includes a servo amplifier 91 that controls the first servo motor 61 rotating the first belt 5, a servo amplifier 92 that controls the second servo motor 79 displacing the first belt 5, and an arithmetic processor 93.

The arithmetic processor 93 outputs operation control signals, which correspond to a rotation pattern of the belt and a displacement pattern of the belt, to the servo amplifiers 91 and 92 on the basis of the rotation pattern of the belt and the displacement pattern of the belt, which are preset, and a reference signal 95 that is generated from a rotary encoder provided in a facility, for example, a rotary encoder connected to a rotary cutter or the like cutting a product one by one, continuously changes, and is reset whenever one product is manufactured. The product is a product, such as a composite sheet that is obtained using the apparatus 1 or an absorbent article (a disposable diaper 10 or the like) that is manufactured by introducing the composite sheet obtained by the apparatus 1 to a manufacturing line.

The rotation pattern of the belt or the displacement pattern of the belt may be input to the arithmetic processor 93 from an operating unit (not illustrated), and may be input to the arithmetic processor 93 through a communication line or a storage medium such as a USB memory. Further, the rotation pattern of the belt and the displacement pattern of the belt may be input to the arithmetic processor 93, respectively. Alternatively, an oscillating pattern of the oscillating unit (a combined pattern of the rotation pattern of the belt and the displacement pattern of the belt) may be input to the arithmetic processor 93 and the rotation pattern of the belt and the displacement pattern of the belt may be calculated from the oscillating pattern according to a separately input distribution rule or a preset distribution rule. Furthermore, a signal, which is generated by a controller controlling a facility in which the oscillating/feeding apparatus 1 is installed, may be input to the arithmetic processor 93 as the reference signal, and the reference signal may be generated in the arithmetic processor 93. A processor, which is obtained by adding various interfaces to a programmable logic controller (PLC) or a commercially available personal computer, or the like can be used as the arithmetic processor 93.

Next, an example of a method of manufacturing an extensible sheet by using the above-mentioned oscillating/feeding apparatus 1 for an elastic member will be described.

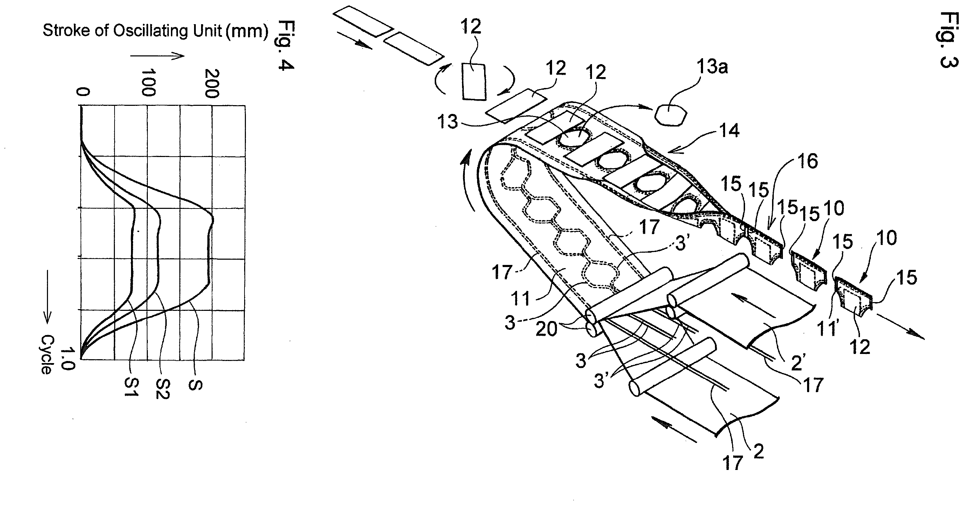

As illustrated in Figs. 2 and 3, in a method of manufacturing an extensible sheet of this embodiment, an elastic member 3 is continuously fed onto a continuously conveyed continuous sheet 2 while the elastic member 3 is oscillated in a direction intersecting a traveling direction of the sheet 2 by the above-mentioned oscillating/feeding apparatus 1 for an elastic member, and an elastic member 3' is continuously fed onto a continuously conveyed continuous sheet 2' while the elastic member 3' is oscillated in a direction intersecting a traveling direction of the sheet 2' by a second oscillating/feeding apparatus (an oscillating unit 8' and a first belt 5' to which the oscillating unit 8' is fixed are illustrated in Fig. 2) having the same structure as the structure of the above-mentioned oscillating/feeding apparatus for an elastic member (feeding step). Further, the elastic members 3 and 3' and the upper and lower sheets 2 and 2' are integrated with each other by being pinched and pressed by a pair of rollers 20 and 20 (integration step) while the elastic members 3 and 3' are interposed between the sheets 2 and 2'. Accordingly, a continuous outer cover 11 of a pull-on disposable diaper 10 is obtained as an extensible sheet 11.

It is preferable that the oscillating/feeding apparatus 1 for an elastic member and the second oscillating/feeding apparatus be installed so as to face each other with a common contact surface S between the rollers 20 and 20 interposed therebetween. The second oscillating/feeding apparatus may also include the same arithmetic processor as the arithmetic processor 93 of the oscillating/feeding apparatus 1 for an elastic member, and both the oscillating/feeding apparatus 1 and the second oscillating/feeding apparatus may be controlled by one arithmetic processor 93.

Preferably, the sheet 2 onto which the elastic member 3 has not yet been fed is coated with an adhesive by an adhesive coating device (not illustrated), and the sheet 2' onto which the elastic member 3' has not yet been fed is also coated with an adhesive by an adhesive coating device (not illustrated). Each of the adhesive coating devices may be a non-contact type coating device or a contact type coating device. A device or a method, a coating pattern, and the like, which can fix the elastic members 3 and 3' between both the sheets 2 and 2' so that a predetermined curve shape given by the oscillation of the elastic members can be kept, can be used as a device or a method, a coating pattern, and the like, which coat the sheet 2 and/or sheet 2' with an adhesive, without particular limitation. It is preferable that the elastic member 3 in a stretched state be introduced on the sheet 2 or between the sheets 2 and 2', and it is preferable that the elastic member 3' in a stretched state be introduced on the sheet 2' or between the sheets 2 and 2'.

Further, after the elastic member 3 is fed onto the sheet 2 that is not coated with an adhesive, the sheet 2 and the sheet 2' coated with an adhesive may be superimposed and integrated with each other. Likewise, after the elastic member 3' is fed onto the sheet 2' that is not coated with an adhesive, the sheet 2' and the sheet 2 coated with an adhesive may be superimposed and integrated with each other. Furthermore, both the sheets 2 and 2' are not coated with an adhesive, and only any one of the sheets 2 and 2' may be coated with an adhesive. Moreover, one sheet is completely coated and only a portion of the other sheet, which requires a particularly strong adhesive force, is intermittently coated with an adhesive, that is, one sheet and the other sheet can be made to have different coating patterns of an adhesive.

The pull-on disposable diaper 10 can be manufactured in the same method as a well-known method of manufacturing a so-called lateral flow type pull-on disposable diaper except that the continuous outer cover 11 obtained as described above is used. For example, it is possible to obtain a pull-on disposable diaper 10, which includes an outer cover 11' formed of a cut continuous outer cover 11, by sequentially performing a step of intermittently and sequentially disposing and fixing absorbent assemblies 12 to the continuous outer cover 11 which is obtained in a step of manufacturing an extensible sheet, a step of forming openings 13 for forming leg openings in the continuous outer cover 11, a step of folding a continuous body 14 in which the openings have been formed in a width direction, a step of forming side seals 15 and 15 at the folded continuous body, and a step of cutting a diaper continuous body 16, which has been obtained as described above, into dimensions of each diaper 10, as illustrated in Fig. 3.

Meanwhile, the absorbent assembly 12 of the pull-on disposable diaper 10 includes a liquid permeable topsheet (not illustrated) that forms a skin-facing surface, a liquid impermeable or water repellent leakage-preventive sheet (not illustrated), and a liquid retentive absorbent member (not illustrated) that is disposed between the topsheet and the leakage-preventive sheet.

Further, reference numeral 13a of Fig. 3 denotes trims that are removed to form the openings 13 for forming leg openings.

Furthermore, in the step of manufacturing the extensible sheet in the embodiment illustrated in Fig. 3, elastic members 17 and 17, which form waist gathers at the peripheral portion of a waist opening, are introduced in the traveling direction of the sheets 2 and 2' without oscillating. As described above, the extensible sheet manufactured in the invention may include the elastic member 17, which is introduced without oscillating, in addition to the elastic member 3 that is introduced while oscillating. Further, the step of forming the openings may be performed before the step of disposing the absorbent assemblies, and the step of forming the openings and the step of disposing the absorbent assemblies may be performed at the same time.

In Fig. 4, a manufacturing cycle of one product is represented by a horizontal axis. Fig. 4 illustrates a displacement pattern of the oscillating unit 8, that is, an oscillating pattern S, a displacement pattern of the oscillating unit 8 formed by the rotation of the first belt 5, that is, a rotation pattern S1, and a displacement pattern of the oscillating unit 8 formed by the displacement of the first belt 5 caused by the second displacement means, that is, a displacement pattern S2. In this apparatus, the oscillating pattern S, which is obtained by the disposition shape of the elastic member 3 on the product, is formed by the combination of the rotation pattern S1 and the displacement pattern S2. Accordingly, a moving distance, speed, and acceleration, which are caused by the first (rotation) servo motor and the second (displacement) servo motor, can be reduced as compared to the oscillating pattern S. Here, as for the distribution of the rotation pattern S1 and the displacement pattern S2, each of the rotation pattern S1 and the displacement pattern S2 may be distributed as 50% of the oscillating pattern S. However, speed and acceleration where the servo motors can rotate vary according to the capacities or inertia of the first and second servo motors or the inertia of parts, such as pulley or guides connected to the respective servo motors. Accordingly, in this apparatus, it is possible to efficiently speed up the apparatus by changing a balance of the rotation pattern S1 and the displacement pattern S2, for example, by combining the load factors of the respective servo motors. Examples of a preferred embodiment include an embodiment in which each of the rotation pattern S1 and the displacement pattern S2 is distributed as 50% of the oscillating pattern S or the rotation pattern S1 and the displacement pattern S2 are distributed so that the load factors of the respective servo motors correspond to each other.

The specific oscillation of the oscillating unit 8 will be described with reference to Fig. 1. When the oscillating unit 8 is displaced from one end E1 of the oscillating range (amplitude) W thereof toward the other end E2, the driving pulley 43 is rotated in the direction of an arrow a to rotate the first belt 5 in a clockwise direction and the sliders 71 and 72 are displaced in the direction of an arrow a' to move the position of the first belt 5 (particularly, a portion of the first belt 5 to which the oscillating unit 8 is fixed) in the direction of an arrow A. On the other hand, when the oscillating unit 8 is displaced from one end E2 of the oscillating range (amplitude) W thereof toward the other end E1, the driving pulley 43 is rotated in the direction of an arrow b to rotate the first belt 5 in a counterclockwise direction and the sliders 71 and 72 are displaced in the direction of an arrow b' to displace the first belt 5 (particularly, a portion of the first belt 5 to which the oscillating unit 8 is fixed) in the direction of an arrow B.

The second oscillating/feeding apparatus for an elastic member can be formed so as to have the same structure as the structure of the oscillating/feeding apparatus 1 for an elastic member except that the oscillating unit is oscillated so that the elastic member 3' draws a curved line, which is substantially symmetrical to the elastic member 3 with respect to a straight line along the longitudinal direction of the extensible sheet 11 as illustrated in Fig. 3.

Further, in Fig. 4, the rotation pattern S1 and the displacement pattern S2 have been distributed for the oscillating pattern S at a constant ratio. However, the ratio of the load factors may be changed within a cycle in order to efficiently share a load factor.

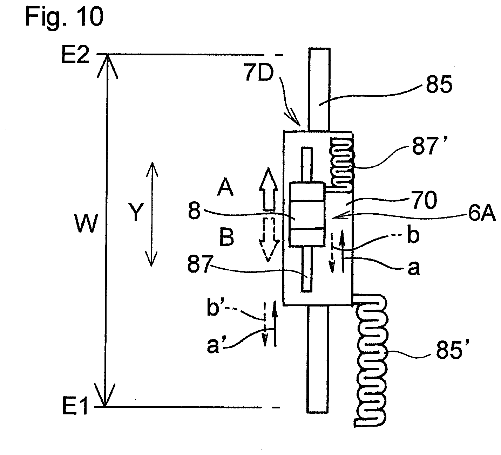

Meanwhile, the first displacement means of the invention is not limited to a belt rotation means that displaces an oscillating unit by rotating a belt to which the oscillating unit is fixed, similar to a first displacement means 6A of an apparatus illustrated in Fig. 10. Even though the first displacement means is not the belt rotation means, it is preferable that a composite sheet be manufactured while control is performed so that the oscillating pattern S of the oscillating unit is formed by the combination of the rotation pattern S1 formed by the first displacement means and the displacement pattern S2 formed by the second displacement means. Further, likewise, it is also preferable that a displacement pattern S1 formed by the first displacement means and the displacement pattern S2 formed by the second displacement means be distributed so that the load factor of a servo motor or a linear motor as a driving source of the first displacement means corresponds to the load factor of a servo motor or a linear motor as a driving source of the second displacement means.

According to the oscillating/feeding apparatus 1 for an elastic member of the above-mentioned embodiment and the method of manufacturing the extensible sheet 11 using the apparatus, the reciprocation (oscillation) of the oscillating unit 8 is performed by the cooperation of a means that rotates the first belt 5 and a means that moves the position of the first belt 5. Accordingly, the operational speed (rotational speed), the torque, or the like of each of the driving sources can be reduced as compared to, for example, a case in which the reciprocation (oscillation) of the oscillating unit 8 is performed by only the rotation of the first belt 5. For this reason, since it is possible to increase the movement speed or the acceleration of the oscillating unit while controlling the load of the driving source, it is easier to speed up a facility or to change of a curve shape in which the elastic member is disposed.

It is preferable that the belt rotation means 6 and the belt displacement means 7 be driven by the servo motors 61 and 79 as in this embodiment since the position of the oscillating unit 8 can be more accurately controlled.

Further, it is preferable that the belt displacement means 7 include the plurality of sliders 71 and 72 including the driven pulleys 41 and 42 engaged with the first belt 5, and the driving source (servo motor 79 or the like) moving the positions of the sliders 71 and 72 as in this embodiment in terms of the fact that the size of a device that should be moved to displace the first belt 5 can be reduced as much as possible and the movement speed or the acceleration of the oscillating unit can be further increased.

Furthermore, it is preferable that the driving source moving the positions of the sliders 71 and 72 be a servo motor in terms of the fact that the position of the oscillating unit 8 can be more accurately controlled. Moreover, it is preferable that a linear motor enabling positioning be used in terms of the simplification of a mechanism.

Meanwhile, one driving source (servo motor or the like) may be provided for a plurality of sliders as the driving source that displaces the plurality of sliders 71 and 72 as in this embodiment, and one driving source may be provided for each of the sliders as in a second embodiment to be described below.

Further, the displacements of both the sliders 71 and 72 interlock with each other in the apparatus 1 according to this embodiment since the second belt 75 joined to the plurality of sliders 71 and 72 is driven by the servo motor 79. Since the displacements of both the sliders 71 and 72 interlock with each other, it is possible to accurately control the oscillation of the oscillating unit 8.

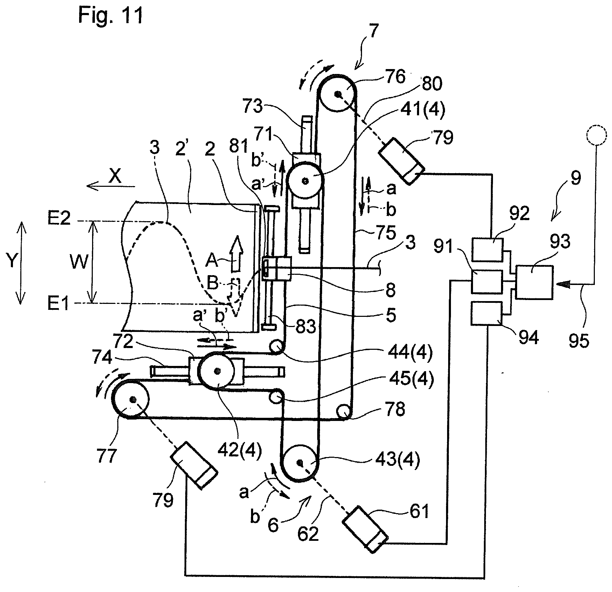

Furthermore, when a servo motor 79 is also installed on the pulley 77 as illustrated in Fig. 11 and the belt is displaced by the combination of the servo motor 79 of the pulley 76 and a plurality of servo motors, it is possible to accurately displace the belt since the sliders 71 and 72 are mechanically connected to each other, for example, even though the inertia or frictional resistance of the slider 71 is different from the inertia or frictional resistance of the slider 72.

Fig. 11 is a view illustrating a modification of the first embodiment and the same components as the components of the apparatus illustrated in Fig. 1 are denoted by the same reference numerals. An apparatus illustrated in Fig. 11 includes the servo motor 79 that drives the pulley 77, and has the same structure as the structure of the apparatus illustrated in Fig. 1 except that the pulley 77 is rotated in the same pattern as the pattern of the pulley 76 by the control unit 9.

Moreover, in the apparatus 1 of the first embodiment (the modification of the first embodiment illustrated in Fig. 11 and a second embodiment to be described below are also the same), the sliding direction of the slider 71 is made to be parallel to the oscillation direction (Y direction) of the oscillating unit 8 and the sliding direction of the slider 72 is made to be substantially orthogonal to the oscillation direction of the oscillating unit 8. Since the plurality of sliders 71 and 72 of which the sliding directions are different from each other are provided as described above, it is possible to save a space in which the apparatus or the invention is embodied.

Next, other embodiments of the invention will be described. Portions of other embodiments, which are different from the first embodiment, will be described, and the same portions of other embodiments as the first embodiment are denoted by the same reference numerals and the description thereof will be omitted. The description of the above-mentioned first embodiment will be appropriately applied to portions that are not particularly described.

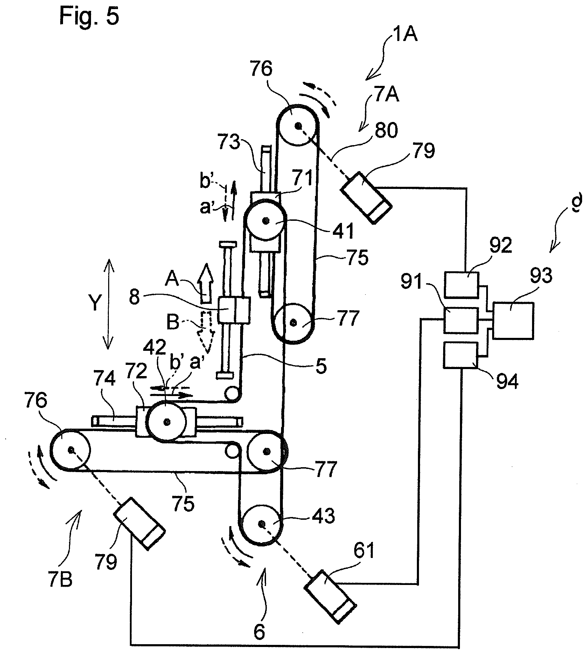

As illustrated in Fig. 5, an oscillating/feeding apparatus for an elastic member according to a second embodiment includes slider driving means 7A and 7B that are provided for the sliders 71 and 72, respectively, as a belt displacement means moving the positions of a plurality of sliders 71 and 72 to move the position of a first belt 5 (particularly, a portion of the first belt 5 to which an oscillating unit 8 is fixed) in a Y direction. Each of the slider driving means 7A and 7B includes an endless second belt 75 that is fixed to the slider, and a driving pulley 76 and a driven pulley 77 between which the second belt 75 is bridged. Further, the driving pulley 76 is rotationally driven by a second servo motor 79, so that the second belt 75 and the sliders 71 and 72 joined to the second belt 75 are displaced in an a' or b' direction of Fig. 5.

In the second embodiment, an arithmetic processor 93 of a control unit 9 makes the displacements of both the sliders 71 and 72 interlock with each other by outputting operation control signals, which operate the second servo motor 79 in synchronization, to servo amplifiers 92 and 94. That is, when one slider 71 is displaced in the a' direction, the other slider 72 is also displaced in the a' direction at the same speed. When one slider 71 is displaced in the b' direction, the other slider 72 is also displaced in the b' direction at the same speed.

Accordingly, since a load per second servo motor 79 can be reduced, it is possible to speed up the belt displacement means. Further, since the second belt 75 can be made short, the meandering or bend of the belt can be suppressed.

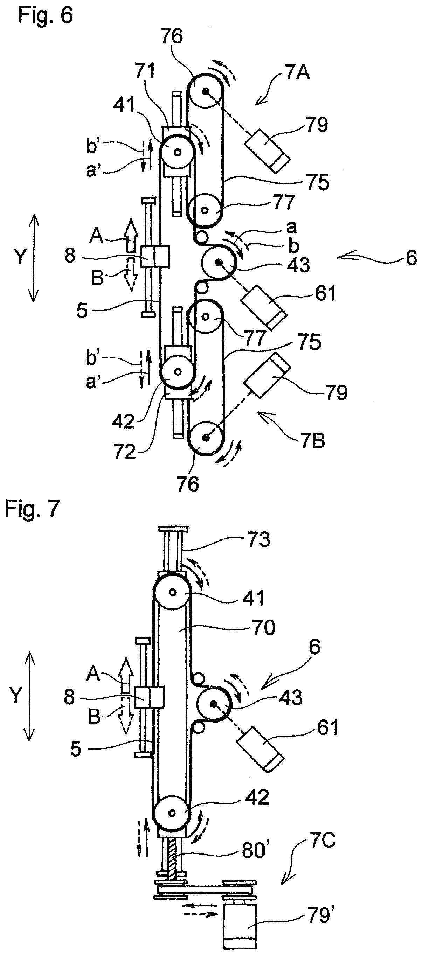

As illustrated in Fig. 6, an oscillating/feeding apparatus for an elastic member according to a third embodiment includes the slider driving means 7A and 7B in which a belt displacement means (second displacement means) moving the positions of a plurality of sliders 71 and 72 to move the position of a first belt 5 (particularly, a portion of the first belt 5 to which an oscillating unit 8 is fixed) in a Y direction is provided for each of the sliders 71 and 72, respectively. Further, both the sliding direction of the slider 71, which is driven by one slider driving means 7A, and the sliding direction of the slider 72, which is driven by the other slider driving means 7B, are the intersecting direction (Y direction) intersecting a traveling direction of a sheet. Furthermore, a driving pulley 76 is rotationally driven by a second servo motor 79, so that a second belt 75 and the slider 71 or 72 joined to the second belt 75 are displaced in an a' or b' direction of Fig. 6. Meanwhile, a belt rotation means 6 (first displacement means) of the third embodiment also includes a well-known power transmission mechanism that is formed by the combination or the like of a first servo motor 61 as a driving source and gears transmitting the rotation of the motor 61 to a driving pulley 43. The slider driving means 7A and 7B form a belt displacement means (second displacement means).

Even in the third embodiment, the displacements of both the sliders 71 and 72 are made to interlock with each other by the same method as the method of the second embodiment. It is possible to save a space in the traveling direction of a sheet in addition to the fact that the same effect as the effect of the second embodiment is obtained as compared to the first embodiment.

As illustrated in Fig. 7, in an oscillating/feeding apparatus for an elastic member according to a fourth embodiment, a plurality of driven pulleys 41 and 42 between which a first belt 5 is bridged are rotatably provided on a moving stand 70 that is displaced along slide guides 73 in an intersecting direction Y intersecting the traveling direction of a sheet 2, and the first belt 5 is rotationally driven by a driving pulley 43 that is disposed beside the moving stand 70.

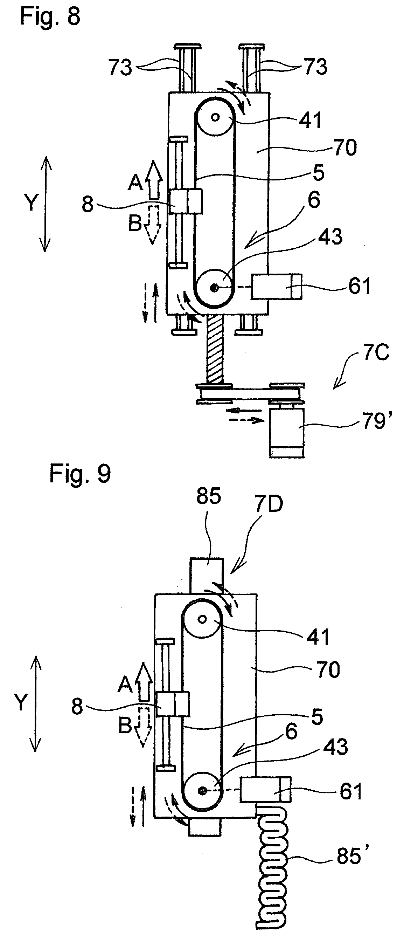

As illustrated in Fig. 8, in an oscillating/feeding apparatus for an elastic member according to a fifth embodiment, a plurality of pulleys 41 and 43 between which a first belt 5 is bridged and a servo motor 61 that drives a pulley (driving pulley) 43 to rotate the first belt 5 are provided on a moving stand 70 that is displaced along the slide guides 73 in an intersecting direction Y intersecting the traveling direction of a sheet 2.

In the fourth and fifth embodiments, a belt displacement means 7C (second displacement means) includes the above-mentioned moving stand 70, a third servo motor 79' that displaces the moving stand 70 in the Y direction, and the like. The torque of the third servo motor 79' is transmitted to a ball screw 80' by an arbitrary power transmission mechanism, such as a pulley or a gear, and the ball screw 80' is rotated while being threadably engaged with a female screw portion (not illustrated) provided at the moving stand 70, so that the moving stand 70 is displaced in the Y direction.

Even in the fourth and fifth embodiments, the oscillating unit 8 is oscillated by the cooperation of the movement of the position of the first belt 5, which is caused by the displacement of the moving stand 70, and the rotation of the first belt 5, so that the same effect as the effect of the first embodiment is obtained. It is possible to more accurately displace the belt by using a ball screw in the belt displacement means.

In an oscillating/feeding apparatus for an elastic member according to a sixth embodiment, as illustrated in Fig. 9, a plurality of pulleys 41 and 43 between which a first belt 5 is bridged and a servo motor 61 that drives the driving pulley 43 of the pulleys to rotate the first belt 5 are provided on a moving stand 70 that is displaced in an intersecting direction Y intersecting the traveling direction of a sheet 2 by a linear motor 85. The oscillating unit 8 is oscillated by the cooperation of a belt rotation means 6 (first displacement means) that is driven by the servo motor 61 and a belt displacement means 7D (second displacement means) that moves the position of the first belt 5 by the linear motor 85, so that the same effect as the effect of the first embodiment is obtained. Further, since the linear motor is used, the structure of a mechanism can be made simple and the number of parts is reduced. Accordingly, maintenance work can be reduced.

Meanwhile, a commercially available linear motor, which includes a movable element including coils and a stator including a magnet, or the like can be used as the linear motor. For example, a movable element is provided on the back of the moving stand 70 and a stator is disposed between slide guides 73 that face the back of the moving stand 70. In Figs. 9 and 10, reference numerals 85' and 87' denote movable cables that supply current to coils of a linear motor.

As illustrated in Fig. 10, in an oscillating/feeding apparatus for an elastic member according to a seventh embodiment, an oscillating unit 8, which is displaced in an intersecting direction Y intersecting the traveling direction of a sheet 2 by a linear motor 87, is provided on a moving stand (moving body) 70 that is displaced in the intersecting direction Y intersecting the traveling direction of the sheet 2 by a linear motor 85. The oscillating unit 8 is oscillated by the cooperation of the first and second linear motors 85 and 87, so that the same effect as the effect of the first embodiment is obtained. A first displacement means 6A of this embodiment includes the second linear motor 87 that oscillates the oscillating unit 8 on the moving stand 70 and slide guides (not illustrated) that regulate the sliding direction of the oscillating unit 8 on the moving stand 70 into a Y direction illustrated in Fig. 10. The second displacement means 7D includes the first linear motor 85 that displaces the moving stand 70 and slide guides (not illustrated) that regulate the sliding direction of the moving stand 70 in the Y direction illustrated in Fig. 10.

Even in the seventh embodiment, when the oscillating unit 8 is displaced from one end E1 of the oscillating range (amplitude) thereof toward the other end E2, the oscillating unit 8 is displaced in the a direction along the intersecting direction on the moving stand 70 by the second linear motor 87 and the moving stand 70 itself is displaced in the a' direction along the intersecting direction Y by the first linear motor 85. On the other hand, when the oscillating unit 8 is displaced from one end E2 of the oscillating range (amplitude) W thereof toward the other end E1, the oscillating unit 8 is displaced in the b direction along the intersecting direction on the moving stand 70 by the second linear motor 87 and the moving stand 70 itself is displaced in the b' direction along the intersecting direction Y by the first linear motor 85.

According to the oscillating/feeding apparatus for an elastic member according to the seventh embodiment and a method of manufacturing an extensible sheet by using the oscillating/feeding apparatus, the reciprocation (oscillation) of the oscillating unit 8 is achieved by the cooperation of the first and second linear motors. Accordingly, it is possible to reduce the operational speed, the acceleration, and the like of both the motors, and to speed up the oscillating unit as compared to a case in which the oscillating unit 8 is displaced by one linear motor. Further, since the linear motor is used as in the sixth embodiment, the structure of a mechanism can be made simple and the number of parts is reduced. Accordingly, maintenance work can be reduced.

The invention is not limited to the above-mentioned embodiments, and may be appropriately modified without departing from the scope of the invention.

For example, the elastic member, which is supplied as a continuous member, may be a composite elastic member in which a rubber thread or the like is fixed to one surface of a narrow sheet-like member or between sheets in a stretched state, a narrow elastic film, or the like other than a filamentous or narrow continuous elastic member (rubber thread, flat rubber, or the like). Further, the continuous member is not limited to the elastic member, and may be an inelastic filamentous member (color yarn or the like), an inelastic narrow sheet-like member, or the like. The width of the narrow sheet-like member is preferably equal to or smaller than the width of a continuous sheet to which the narrow sheet-like member is supplied while being oscillated, is more preferably in the range of 10 to 50 mm, and is even more preferably in the range of 15 to 30 mm.

Furthermore, since the continuous member may not be an elastic member, the composite sheet manufactured by a method of manufacturing a composite sheet of the invention may be an inextensible composite sheet.

Moreover, the extensible sheet may include only one sheet and a single or a plurality of elastic members fixed to one surface of the sheet. Further, the extensible sheet may include only one fixed elastic member. Furthermore, when an extensible sheet where a plurality of elastic members are fixed between sheets is obtained, all of the elastic members may be introduced so as to draw curved lines parallel to each other.

Moreover, when a continuous outer cover of a pull-on wearing article is manufactured as the extensible sheet, the pull-on wearing article may be a shorts-type napkin or the like instead of a disposable diaper.

The curved shapes of the first and second elastic members are not limited to the curved shapes illustrated in Figs. 2 and 3, respectively, and may be the shape of, for example, a pure sine curve.

Further, an example where one elastic member 3 is introduced so as to come into contact with the sheet 2 first, and the other elastic member 3' is introduced so as to come into contact with the first sheet 2' first has been described in the above-mentioned embodiments, but both the elastic members 3 and 3' may be introduced so as to come into contact with the same sheet first.

The invention is an oscillating/feeding apparatus according to claims 1-6, and methods of manufacturing a composite sheet and a diaper by means of this apparatus, according to claims 7-12.

### Industrial Applicability

According to the oscillating/feeding apparatus for a continuous member and a method of manufacturing a composite sheet of the invention, it is possible to displace an oscillating unit at a high speed or high acceleration while reducing the speed or acceleration of a motor. For this reason, for example, it is possible to speed up facilities that manufacture various products using the continuous member or to increase the speed or acceleration of an oscillating unit by the change of a curve shape in which the continuous member is disposed.

## Claims

1. An oscillating/feeding apparatus (1) for a continuous member that feeds a continuous member (3) onto a continuously conveyed continuous sheet (2) while oscillating the continuous member in an intersecting direction (Y) intersecting a traveling direction of the sheet (2),
the apparatus comprising:
an oscillating unit (8) that is oscillated in the intersecting direction (Y);
a first displacement means (6) that displaces and oscillates the oscillating unit (8) in the intersecting direction (Y);
a second displacement means (7) that displaces and oscillates the oscillating unit (8) in the intersecting direction (Y) by displacing a part or all of the first displacement means (6) in the intersecting direction (Y); and
a control unit (9) that controls the first displacement means (6) and second displacement means (7),
the oscillating unit (8) including an outlet (81) which feeds the continuous member (3) to the sheet (2);
wherein the second displacement means (7) is connected to the first displacement means (6);
wherein each of the first displacement means (6) and second displacement means (7) includes a driving source (61) that is formed of one or more servo motors (61, 79) or linear motors (85, 87);
wherein the oscillating unit (8) includes two through holes that are formed at a block-like fixed part (82) fixed to an endless belt (5), and is oscillated along two slide shafts (83) that are inserted into the through holes, respectively.

2. The oscillating/feeding apparatus (1) for a continuous member according to claim 1,
wherein the control unit (9) controls the first displacement means (6) and second displacement means (7) according to a reference signal, and controls the oscillation of the oscillating unit (8).

3. The oscillating/feeding apparatus (1) for a continuous member according to claim 2,
wherein the first displacement means (6) includes the endless belt (5) to which the oscillating unit (8) is fixed, a plurality of pulleys (4) that are engaged with the belt, and one or more driving sources that are connected to the one or more pulleys.

4. The oscillating/feeding apparatus (1) for a continuous member according to claim 3,
wherein the second displacement means (7) includes a plurality of sliders (71, 72) that include the pulleys engaged with the belt, respectively, a means that connects the plurality of sliders (71, 72), and one or more driving sources that displace the plurality of connected sliders (71, 72).

5. The oscillating/feeding apparatus (1) for a continuous member according to claim 4,
wherein the second displacement means (7) includes a plurality of sliders (71, 72) that include the pulleys engaged with the belt, respectively, and one or more driving sources that move the positions of the sliders (71, 72), respectively.

6. The oscillating/feeding apparatus for a continuous member according to any one of claims 1 to 2,
wherein the first displacement means (6) is a belt rotation means that rotates the endless belt (5) bridged between a plurality of pulleys (4),
the second displacement means (7) is a belt displacement means that displaces the belt (5) in the intersecting direction (Y) intersecting the traveling direction (X) of the sheet (2), and
the oscillating unit (8) is oscillated in the intersecting direction (Y) with the rotation and displacement of the belt (5).

7. A method of manufacturing a composite sheet in which a continuous member is fixed to a sheet in a curved shape,
the method comprising:
a feeding step of continuously feeding the continuous member onto a continuously conveyed sheet,
wherein in the feeding step, the continuous member is fed while being oscillated in a direction intersecting the traveling direction of the sheet by the oscillating/feeding apparatus (1) for a continuous member according to any one of claims 1 to 6.

8. The method of manufacturing a composite sheet according to claim 7,
wherein in the feeding step, a first continuous member is continuously fed onto a continuous first sheet (2'), which is continuously conveyed, while being oscillated in a direction intersecting a traveling direction of the first sheet (2') by a first oscillating/feeding apparatus, and a second continuous member is continuously fed onto a continuous second sheet, which is continuously conveyed, while being oscillated in a direction intersecting a traveling direction of the second sheet by a second oscillating/feeding apparatus (1) that has the same structure as the structure of the first oscillating/feeding apparatus.

9. The method of manufacturing a composite sheet according to claim 7 or 8,
wherein the continuous member is an elastic member (3), and
the composite sheet manufactured is an extensible sheet (11) including the sheet and the elastic member (3) which is fixed in a stretched state to the sheet.

10. The method of manufacturing a composite sheet according to any one of claims 7 to 9,
wherein the composite sheet is manufactured while control is performed so that an oscillating pattern S of the oscillating unit (8) is formed by the combination of a displacement pattern S1 formed by the first displacement means (6) and a displacement pattern S2 formed by the second displacement means (7).

11. The method of manufacturing a composite sheet according to any one of claims 7 to 10,
wherein driving sources of the first displacement means (6) and second displacement means (7) are servo motors, and
the displacement pattern S1 formed by the first displacement means (6) and the displacement pattern S2 formed by the second displacement means (7) are distributed so that load factors of both the servo motors correspond to each other.

12. A method of manufacturing a pull-on disposable diaper (10) by using a composite sheet, which is obtained by the method of manufacturing a composite sheet according to any one of claims 7 to 11, as a continuous outer cover,
the method comprising:
a step of intermittently and sequentially disposing and fixing absorbent assemblies (12) to the continuous outer cover (11) which is obtained in a step of manufacturing an extensible sheet (11);
a step of forming openings (13) for forming leg openings in the continuous outer cover (11);
a step of folding the continuous outer cover (11) in which the openings have been formed in a width direction;
a step of forming side seals (15) at the folded continuous outer cover (11); and
a step of cutting a diaper continuous body (16), at which the side seals (15) have been formed, into each pull-on disposable diaper (10).

## Patentansprüche

1. Schwingungs-/Zuführungsvorrichtung (1) für ein kontinuierliches Element, die ein kontinuierliches Element (3) auf eine kontinuierlich beförderte kontinuierliche Bahn (2) zuführt, während sie das kontinuierliche Element in eine Querrichtung (Y), die die Bewegungsrichtung der Bahn (2) schneidet, in Schwingungen versetzt,
wobei die Vorrichtung aufweist:
eine Schwingungseinheit (8), die in die Querrichtung (Y) in Schwingungen versetzt wird;
ein erstes Verschiebungsmittel (6), das die Schwingungseinheit (8) in die Querrichtung (Y) verschiebt und in Schwingungen versetzt;
ein zweites Verschiebungsmittel (7), das die Schwingungseinheit (8) in die Querrichtung (Y) verschiebt und in Schwingungen versetzt, indem es einen Teil oder die Gesamtheit des ersten Verschiebungsmittels (6) in die Querrichtung (Y) verschiebt; und
eine Steuereinheit (9), die das erste Verschiebungsmittel (6) und das zweite Verschiebungsmittel (7) steuert,
wobei die Schwingungseinheit (8) einen Auslass (81) aufweist, der das kontinuierliche Element (3) der Bahn (2) zuführt;
wobei das zweite Verschiebungsmittel (7) mit dem ersten Verschiebungsmittel (6) verbunden ist;
wobei das erste Verschiebungsmittel (6) und das zweite Verschiebungsmittel (7) jeweils eine Antriebsquelle (61) aufweisen, die aus einem oder mehreren Servomotoren (61, 79) oder Linearmotoren (85, 87) gebildet wird;
wobei die Schwingungseinheit (8) zwei Durchgangslöcher aufweist, die an einem blockförmigen befestigten Teil (82) ausgebildet sind, der an einem Endlosband (5) befestigt ist, und längs zweier Gleitschäfte (83) in Schwingungen versetzt wird, die jeweils in die Durchgangslöcher eingesetzt sind.

2. Schwingungs-/Zuführungsvorrichtung (1) für ein kontinuierliches Element nach Anspruch 1,
wobei die Steuereinheit (9) das erste Verschiebungsmittel (6) und das zweite Verschiebungsmittel (7) gemäß einem Referenzsignal steuert und die Schwingung der Schwingungseinheit (8) steuert.

3. Schwingungs-/Zuführungsvorrichtung (1) für ein kontinuierliches Element nach Anspruch 2,
wobei das erste Verschiebungsmittel (6) das Endlosband (5), an dem die Schwingungseinheit (8) befestigt ist, mehrere Bandtrommeln (4), die mit dem Band in Eingriff stehen, und eine oder mehrere Antriebsquellen aufweist, die mit der einen oder den mehreren Bandtrommeln verbunden sind.

4. Schwingungs-/Zuführungsvorrichtung (1) für ein kontinuierliches Element nach Anspruch 3,
wobei das zweite Verschiebungsmittel (7) mehrere Gleitstücke (71, 72), die jeweils die Bandtrommeln aufweisen, die mit dem Band in Eingriff stehen, ein Mittel, das die mehreren Gleitstücke (71, 72) verbindet, und eine oder mehrere Antriebsquellen aufweist, die die mehreren verbundenen Gleitstücke (71, 72) verschieben.

5. Schwingungs-/Zuführungsvorrichtung (1) für ein kontinuierliches Element nach Anspruch 4,
wobei das zweite Verschiebungsmittel (7) mehrere Gleitstücke (71, 72), die jeweils die mit dem Band in Eingriff befindlichen Bandtrommeln aufweisen, und eine oder mehrere Antriebsquellen aufweist, die jeweils die Positionen der Gleitstücke (71, 72) bewegen.

6. Schwingungs-/Zuführungsvorrichtung für ein kontinuierliches Element nach einem der Ansprüche 1 bis 2,
wobei das erste Verschiebungsmittel (6) ein Bandrotationsmittel ist, das das Endlosband (5) dreht, das zwischen mehreren Bandtrommeln (4) eine Brücke bildet,
das zweite Verschiebungsmittel (7) ein Bandverschiebungsmittel ist, das das Band (5) in die Querrichtung (Y) verschiebt, die die Bewegungsrichtung (X) der Bahn (2) schneidet, und
die Schwingungseinheit (8) in die Querrichtung (Y) mit der Rotation und Verschiebung des Bands (5) in Schwingungen versetzt wird.

7. Verfahren zum Herstellen einer Verbundbahn, in der ein kontinuierliches Element an einer Bahn in einer gekrümmten Form befestigt wird,
wobei das Verfahren aufweist:
einen Zuführungsschritt zum kontinuierlichen Zuführen des kontinuierlichen Elements auf eine kontinuierlich beförderte Bahn,
wobei im Zuführungsschritt das kontinuierliche Element zugeführt wird, während es in eine Richtung, die die Bewegungsrichtung der Bahn schneidet, durch die Schwingungs-/Zuführungsvorrichtung (1) für ein kontinuierliches Element nach einem der Ansprüche 1 bis 6 in Schwingungen versetzt wird.

8. Verfahren zum Herstellen einer Verbundbahn nach Anspruch 7,
wobei im Zuführungsschritt ein erstes kontinuierliches Element kontinuierlich auf eine kontinuierliche erste Bahn (2') zugeführt wird, die kontinuierlich befördert wird, während es in eine Richtung, die eine Bewegungsrichtung der ersten Bahn (2') schneidet, durch eine erste Schwingungs-/Zuführungsvorrichtung in Schwingungen versetzt wird, und ein zweites kontinuierliches Element auf eine kontinuierliche zweite Bahn kontinuierlich zugeführt wird, die kontinuierlich befördert wird, während es in eine Richtung, die eine Bewegungsrichtung der zweiten Bahn schneidet, durch eine zweite Schwingungs-/Zuführungsvorrichtung (1) in Schwingungen versetzt wird, die denselben Aufbau wie den Aufbau der ersten Schwingungs-/Zuführungsvorrichtung aufweist.

9. Verfahren zum Herstellen einer Verbundbahn nach Anspruch 7 oder 8,
wobei das kontinuierliche Element ein elastisches Element (3) ist, und
die hergestellte Verbundbahn eine dehnbare Bahn (11) ist, die die Bahn und das elastische Element (3) aufweist, das in einem gedehnten Zustand an der Bahn befestigt wird.

10. Verfahren zum Herstellen einer Verbundbahn nach einem der Ansprüche 7 bis 9, wobei die Verbundbahn hergestellt wird, während eine Steuerung ausgeführt wird, so dass ein Schwingungsmuster S der Schwingungseinheit (8) durch die Kombination eines Verschiebungsmusters S1, das durch das erste Verschiebungsmittel (6) gebildet wird, und eines Verschiebungsmusters S2 gebildet wird, das durch das zweite Verschiebungsmittel (7) gebildet wird.

11. Verfahren zum Herstellen einer Verbundbahn nach einem der Ansprüche 7 bis 10, wobei Antriebsquellen des ersten Verschiebungsmittels (6) und des zweiten Verschiebungsmittels (7) Servomotoren sind, und
das durch das erste Verschiebungsmittel (6) gebildete Verschiebungsmuster S1 und das durch das zweite Verschiebungsmittel (7) gebildete Verschiebungsmuster S2 so verteilt sind, dass Lastfaktoren beider Servomotoren einander entsprechen.

12. Verfahren zum Herstellen einer Wegwerfschlupfwindel (10) durch Verwenden einer Verbundbahn, die durch das Verfahren zum Herstellen einer Verbundbahn nach einem der Ansprüche 7 bis 11 erhalten wird, als eine kontinuierliche äußere Abdeckung, wobei das Verfahren aufweist:
einen Schritt des intermittierenden und aufeinanderfolgenden Anordnens und Befestigens von saugfähigen Anordnungen (12) an der kontinuierlichen äußeren Abdeckung (11), die in einem Schritt zum Herstellen einer dehnbaren Bahn (11) erhalten wird;
einen Schritt des Bildens von Öffnungen (13) zum Bilden von Beinöffnungen in der kontinuierlichen äußeren Abdeckung (11);
einen Schritt des Faltens der kontinuierlichen äußeren Abdeckung (11), in der die Öffnungen in eine Breitenrichtung gebildet worden sind;
einen Schritt des Bildens von seitlichen Abdichtungen (15) an der gefalteten kontinuierlichen äußeren Abdeckung (11); und
einen Schritt des Schneidens eines kontinuierlichen Windelkörpers (16), an dem die seitlichen Abdichtungen (15) ausgebildet worden sind, zu jeder Wegwerfschlupfwindel (10).

## Revendications

1. Dispositif d'oscillation/d'alimentation (1) pour un élément continu, refoulant un élément continu (3) sur une feuille continue transportée en continu (2) en faisant osciller ledit élément continu dans une direction d'intersection (Y) croisant une direction de déplacement de la feuille (2),
ledit dispositif comprenant :
une unité d'oscillation (8) oscillant dans la direction d'intersection (Y) ;
un premier moyen de déplacement (6) déplaçant et faisant osciller l'unité d'oscillation (8) dans la direction d'intersection (Y) ;
un deuxième moyen de déplacement (7) déplaçant et faisant osciller l'unité d'oscillation (8) dans la direction d'intersection (Y) par déplacement d'une partie ou de la totalité du premier moyen de déplacement (6) dans la direction d'intersection (Y) ; et
une unité de commande (9) commandant le premier moyen de déplacement (6) et le deuxième moyen de déplacement (7),
l'unité d'oscillation (8) présentant une sortie (81) refoulant l'élément continu (3) vers la feuille (2) ;
où le deuxième moyen de déplacement (7) est relié au premier moyen de déplacement (6) ;
où le premier moyen de déplacement (6) et le deuxième moyen de déplacement (7) comportent chacun une source d'entraînement (61) constituée d'un ou de plusieurs servomoteurs (61, 79) ou moteurs linéaires (85, 87) ;
où l'unité d'oscillation (8) présente deux trous traversants formés sur une partie fixe en forme de bloc (82) fixée à une courroie sans fin (5), et oscille le long deux tiges coulissantes (83) engagées chacune dans le trou traversant correspondant.

2. Dispositif d'oscillation/d'alimentation (1) pour un élément continu selon la revendication 1,
où l'unité de commande (9) commande le premier moyen de déplacement (6) et le deuxième moyen de déplacement (7) en fonction d'un signal de référence, et commande l'oscillation de l'unité d'oscillation (8).

3. Dispositif d'oscillation/d'alimentation (1) pour un élément continu selon la revendication 2,
où le premier moyen de déplacement (6) comprend la courroie sans fin (5) à laquelle l'unité d'oscillation (8) est fixée, une pluralité de poulies (4) en prise avec la courroie, et une ou plusieurs sources d'entraînement reliés à la, ou aux poulies.

4. Dispositif d'oscillation/d'alimentation (1) pour un élément continu selon la revendication 3,
où le deuxième moyen de déplacement (7) comprend une pluralité de coulisseaux (71, 72) incluant les poulies respectives en prise avec la courroie, un moyen de raccordement de la pluralité de coulisseaux (71, 72), et une ou plusieurs sources d'entraînement déplaçant la pluralité de coulisseaux (71, 72) raccordés.

5. Dispositif d'oscillation/d'alimentation (1) pour un élément continu selon la revendication 4,
où le deuxième moyen de déplacement (7) comprend une pluralité de coulisseaux (71, 72) incluant les poulies respectives en prise avec la courroie, et une ou plusieurs sources d'entraînement déplaçant les positions des coulisseaux (71, 72) respectifs.

6. Dispositif d'oscillation/d'alimentation pour un élément continu selon la revendication 1 ou la revendication 2,
où le premier moyen de déplacement (6) est un moyen de rotation de courroie entraînant en rotation la courroie sans fin (5) tendue entre une pluralité de poulies (4),
le deuxième moyen de déplacement (7) est un moyen de déplacement de courroie déplaçant la courroie (5) dans la direction d'intersection (Y) croisant la direction de déplacement (X) de la feuille (2), et
l'unité d'oscillation (8) oscille dans la direction d'intersection (Y) par rotation et déplacement de la courroie (5).

7. Procédé de fabrication d'une feuille composite où un élément continu est fixé à une feuille de forme incurvée,
ledit procédé comprenant :
une étape d'alimentation de refoulement continu de l'élément continu sur une feuille transportée en continu,
l'élément continu étant refoulé lors de l'étape d'alimentation en étant soumis à oscillation dans une direction d'intersection de la direction de déplacement de la feuille par le dispositif d'oscillation/d'alimentation (1) pour un élément continu selon l'une des revendications 1 à 6.

8. Procédé de fabrication d'une feuille composite selon la revendication 7,
où, lors de l'étape d'alimentation, un premier élément continu est refoulé en continu sur une première feuille continue (2') transportée en continu, en étant soumis à oscillation dans une direction d'intersection d'une direction de déplacement de la première feuille (2') par un premier dispositif d'oscillation/d'alimentation, et un deuxième élément continu est refoulé en continu sur une deuxième feuille continue transportée en continu, en étant soumis à oscillation dans une direction d'intersection d'une direction de déplacement de la deuxième feuille par un deuxième dispositif d'oscillation/d'alimentation (1) présentant une structure identique à la structure du premier dispositif d'oscillation/d'alimentation.

9. Procédé de fabrication d'une feuille composite selon la revendication 7 ou la revendication 8, où l'élément continu est un élément élastique (3), et où
la feuille composite fabriquée est une feuille (11) extensible comprenant la feuille et l'élément élastique (3) fixé à la feuille en état d'étirement.

10. Procédé de fabrication d'une feuille composite selon l'une des revendications 7 à 9,
où la feuille composite est fabriquée, une commande étant exécutée de manière à former un motif d'oscillation S de l'unité d'oscillation (8) par combinaison d'un motif de déplacement S1 formé par le premier moyen de déplacement (6) et d'un motif de déplacement S2 formé par le deuxième moyen de déplacement (7).

11. Procédé de fabrication d'une feuille composite selon l'une des revendications 7 à 10, où les sources d'entraînement du premier moyen de déplacement (6) et du deuxième moyen de déplacement (7) sont des servomoteurs, et où
le motif de déplacement S1 formé par le premier moyen de déplacement (6) et le motif de déplacement S2 formé par le deuxième moyen de déplacement (7) sont distribués de sorte que les facteurs de charge des deux servomoteurs correspondent l'un à l'autre.

12. Procédé de fabrication d'une couche à enfiler jetable (10) au moyen d'une feuille composite obtenue par le procédé de fabrication d'une feuille composite selon l'une des revendications 7 à 11, en tant qu'enveloppe extérieure continue, ledit procédé comprenant :
une étape de mise en place et de fixation intermittentes et séquentielles d'ensembles absorbants (12) sur l'enveloppe extérieure continue (11) obtenue lors d'une étape de fabrication d'une feuille extensible (11) ;
une étape de formation d'ouvertures (13) destinée à former des ouvertures pour les jambes dans l'enveloppe extérieure continue (11) ;
une étape de pliage de l'enveloppe extérieure continue (11) où les ouvertures ont été formées dans le sens de la largeur ;
une étape de formation de joints latéraux (15) sur l'enveloppe extérieure continue (11) pliée ; et
une étape de découpe d'un corps de couche continu (16) sur lequel les joints latéraux (15) ont été formés pour former chaque couche à enfiler jetable (10).
